# EUROPEAN PATENT APPLICATION

(11) **EP 1 723 956 A1**
(43) Date of publication of application: **22.11.2006**
(21) Application number: 05719258.5
(22) Date of filing: 18.02.2005
(51) Int. Cl.: A61K 31/4188, A61P 9/10, C07D 491/107

(54) **PREVENTIVE OR THERAPEUTIC AGENTS FOR SEVERE DIABETIC RETINOPATHY**

(30) Priority: 20.02.2004 JP 2004044813
(71) Applicant: SANWA KAGAKU KENKYUSHO CO., LTD., Nagoya-shi, Aichi 461-8631 (JP)
(72) Inventor: KAKEHASHI, Akihiro, Saitama 3300803 (JP); SAITO, Yuka, 3300803 (JP); MORI, Kana, Saitama 3650054 (JP); MIZUNO, Kuniharu SANWA KAGAKU KENKYUSHO CO., LTD., Aichi 4618631 (JP)
(74) Representative: Müller Fottner Steinecke
(86) International application number: PCT/JP2005/002539
(87) International publication number: WO 2005/079792

(57) **Abstract**

The object of the present invention is to provide a pharmaceutical preparation for preproliferative diabetic retinopathy and/or proliferative diabetic retinopathy, that is, a prophylactic or therapeutic agent for severe diabetic retinopathy. The invention relates to a prophylactic or therapeutic agent for severe diabetic retinopathy, which comprises, as an active ingredient, a hydantoin derivative represented by the following general formula below, particularly (2S,4S)-6-fluoro-2',5'-dioxospiro[chroman- 4,4'-imidazolidine]-2-carboxamide. The pharmaceutical preparation is used mainly as an agent for suppressing development into preproliferative diabetic retinopathy and/or proliferative diabetic retinopathy or as an agent for suppressing development of preproliferative diabetic retinopathy and/or proliferative diabetic retinopathy. 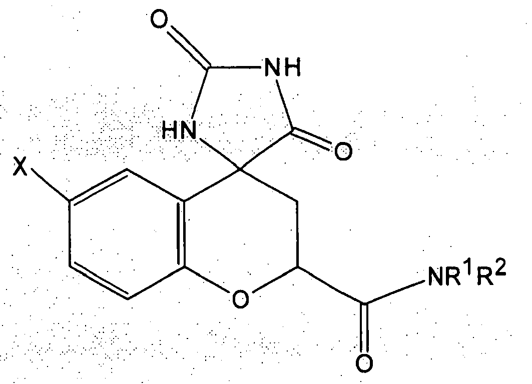 wherein X represents a halogen or a hydrogen atom, R¹ and R² concurrently or differently represent a hydrogen atom or an optionally substituted C1 to C6 alkyl group, or R¹ and R², together with a nitrogen atom bound thereto and optionally another nitrogen atom or an oxygen atom, are combined to form a 5- to 6-membered heterocycle.

## Description

### Technical Field

The present invention relates to new pharmaceutical uses of hydantoin derivatives, particularly (2S,4S)-6-fluoro- 2',5'-dioxospiro[chroman-4,4'-imidazolizine]-2-carboxamid.

### Background Art

As a serious disease among diabetic complications, there is diabetic retinopathy. It is said that about 40% of patients with diabetes mellitus suffer from diabetic retinopathy beginning with early simple retinopathy to become serious in the stage of preproliferative retinopathy and proliferative retinopathy. Particularly problematic in retinopathy is proliferative retinopathy that leads to blindness in the worst case. Since the primary disease causing acquired blindness is proliferative diabetic retinopathy, there is demand for urgent measures against it. It follows that as measures against diabetic retinopathy, there is demand for preventing initial retinopathy from developing into preproliferative retinopathy and proliferative retinopathy or for preventing the progress of preproliferative retinopathy and proliferative retinopathy.

In simple retinopathy, microaneurysm is formed, and by vascular hyperpermeability, hard exudate and retinal edema come to be recognized. In preproliferative retinopathy, retinal vascular disturbance/occlusion causes soft exudate, IRMA (intra-retinal microvascular abnormalities) etc. to occur due to ischemia of retinal tissues. In proliferative retinopathy, neovascularization are newly formed, and vitreous hemorrhage and tractional retinal detachment are recognized thus bringing about serious disorder of visual acuity. In retinopathy, tractional retinal detachment and neovascular glaucoma are 2 major causes of blindness.

In retinopathy, simple retinopathy is generally slow in progress and persists usually for 3 to 10 years. The period of preproliferative retinopathy is said to be half a year to 3 years, and as retinopathy becomes severer, preproliferative retinopathy is assumed to develop into proliferative retinopathy more rapidly in several weeks to several months. From the foregoing, prevention of development of initial retinopathy into preproliferative retinopathy and proliferative retinopathy, or retardation in the progress of preproliferative retinopathy and proliferative retinopathy, is an important key for preventing blindness.

In DCCT Study (large-scale clinical test performed in the US; evaluation of the effect of intensive insulin therapy on type 1 diabetes mellitus) and in Kumamoto Study (clinical test performed in Kumamoto University, Japan; evaluation of the effect of intensive insulin therapy on type 2 diabetes mellitus), strict blood glucose control was revealed to prevent the onset of retinopathy or to inhibit, at some level, the progress of mild retinopathy such as simple retinopathy, that is, mild non-proliferative retinopathy. However, the development and progress of preproliferative retinopathy (i.e., severe non-proliferative retinopathy) and proliferative retinopathy cannot be prevented at present even by strict blood glucose control, and vitreous hemorrhage and retinal detachment cannot be prevented by merely using a hypoglycemic drug.

Some drugs regarded effective for simple retinopathy have been reported, but there is no report on a drug effective for preproliferative retinopathy and proliferative retinopathy. On the other hand, against preproliferative retinopathy and proliferative retinopathy, panretinal photocoagulation is considered effective and applied, but there are many problems such as occurrence of neovascular glaucoma and occurence and worsening of diabetic maculopathy. Vitrectomy for proliferative retinopathy is therapy reported to be effective in many cases, but there are many problems such as a great burden on the patient and still few ophthalmologists who can operate in vitrectomy.

With respect to hydantoin derivatives including (2S,4S)-6-fluoro-2',5'-dioxospiro[chroman-4,4'-imidazolizine]-2-carboxamide found by the applicant, use thereof for diabetic neuropathy is described in JP-A 61-200991, use thereof for circulatory system diseases in JP-A 4-173791, use thereof for various diseases accompanying aging in JP-A 6-135968, use thereof for simple diabetic retinopathy in JP-A 7-242547, and use thereof for diabetic keratopathy in JP-A 8-231549. However, the effectiveness of the hydantoin derivatives for preproliferative diabetic retinopathy and proliferative diabetic retinopathy has not been reported.

As described above, establishment of highly effective therapy for preproliferative diabetic retinopathy and proliferative diabetic retinopathy is strongly desired in the medical field. However, a model for evaluating experimental proliferative diabetic retinopathy, which is important for development of such therapeutic agents, has not been reported so far, and their effectiveness cannot be demonstrated.

As a model showing clinical condition similar to human proliferative retinopathy, a spontaneously diabetic Torii (SDT) rat has been reported in recent years and attracts attention. In a male SDT rat at about 20 weeks of age, there occurs diabetes mellitus, and the rat of advanced age exhibits IRMA (intra-retinal microvascular abnormalities) characteristic of preproliferative retinopathy, retinal hemorrhage characteristic of proliferative retinopathy, and formation of a proliferative neovascular membrane, to cause tractional retinal detachment. It is said that this rat belongs to a model with type II diabetes mellitus because the rat exhibits diabetes mellitus and simultaneously shows hemorrhage and fibrosis of pancreatic Langerhans islet, but does not show an inflammatory change to such a degree as to occur in rats with type I diabetes mellitus such as WBN/Kob rat and BB rat. Since human diabetes mellitus in many cases is type II diabetes mellitus, the effect in this rat will provide significantly important data in consideration of effectiveness against human preproliferative diabetic retinopathy and proliferative diabetic retinopathy.

### Summary of Invention

The present invention was made in consideration of the background described above, and the object of the present invention is to provide a pharmaceutical preparation for preproliferative diabetic retinopathy and/or proliferative diabetic retinopathy, that is, a prophylactic or therapeutic agent for severe diabetic retinopathy.

The present inventors administered (2S,4S)-6-fluoro- 2',5'-dioxospiro[chroman-4,4'-imidazolidine]-2-carboxamide (abbreviated hereinafter as SNK-860) for a long time into SDT rats with proliferative retinal lesions similar to those in humans, to examine its inhibitory effect on the onset. As a result, the inventors revealed that the onset of tractional retinal detachment, the lesions directly leading to blindness, could be strongly inhibited. That is, the present invention relates to a prophylactic or therapeutic agent for severe diabetic retinopathy, which comprises, as an active ingredient, a hydantoin derivative represented by the following formula, preferably (2S,4S)-6-fluoro-2',5'-dioxospiro[chroman- 4,4'-imidazolidine]-2-carboxamide: 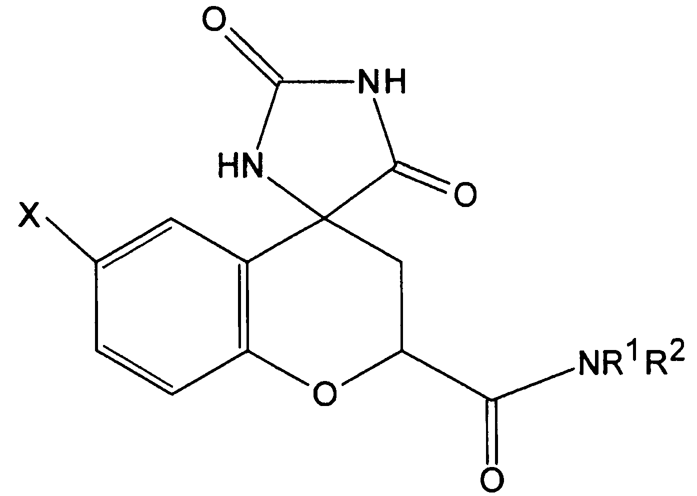 wherein X represents a halogen or a hydrogen atom, R¹ and R² concurrently or differently represent a hydrogen atom or an optionally substituted C1 to C6 alkyl group, or R¹ and R², together with a nitrogen atom bound thereto and optionally another nitrogen atom or an oxygen atom, are combined to form a 5- to 6-membered heterocycle; the halogen represented by X is preferably fluorine, and the C1 to C6 alkyl group is preferably a methyl group.

The prophylactic or therapeutic agent for severe diabetic retinopathy according to the present invention is used mainly as an agent for suppressing development into preproliferative diabetic retinopathy and/or proliferative diabetic retinopathy, or as an agent for suppressing development of preproliferative diabetic retinopathy and/or proliferative diabetic retinopathy.

### Best Mode for Carrying Out the Invention

In the present invention, severe non-proliferative preproliferative diabetic retinopathy and proliferative diabetic retinopathy to which it should be considered to apply panretinal photocoagulation are defined as severe diabetic retinopathy. Hereinafter, the present invention is described in more detail.

Hydantoin derivatives (particularly SNK-860) can be orally administered for example as tablets, capsules, powder, granules, liquid or syrup or parenterally as an injection and suppositories, which were formed by usual pharmaceutical manufacturing techniques. Pharmaceutically acceptable excipients in pharmaceutical manufacturing, for example starch, lactose, refined white sugar, glucose, crystalline cellulose, carboxy cellulose, carboxymethyl cellulose, carboxyethyl cellulose, calcium phosphate, magnesium stearate and gum arabic can be used in the solid preparation, and if necessary a lubricant, a binder, a disintegrating agent, a coating agent, a coloring agent etc. can be incorporated into the solid preparation. In the liquid preparation, a stabilizer, a solubilizer, a suspending agent, an emulsifying agent, a buffer agent, a preservative etc. can be used. The dose varies depending on symptoms, age, administration method, preparation form etc., but preferably the compound described above is administered usually in the range of 1 to 200 mg, preferably 1 to 100 mg, into an adult all at once or in divided portions per day for consecutive days.

### [Pharmacological Study : Example 1]

### 1. Method

Diabetes mellitus occurs in spontaneously diabetic Torii (SDT) rats at about 20 weeks of age. Accordingly, blood glucose levels were confirmed at about 25 weeks of age, and rats showing an increase in blood glucose level (at least 300 mg/dl) were used in the experiment. The male SDT rats confirmed to develop diabetes mellitus were divided into two groups, that is, 1) untreatment control (n = 14) group and 2) SNK-860 treatment (16 mg/kg/day, n = 17)group. The compound was mixed with usual feed (CRF-1: Oriental Yeast Co., Ltd.) and then administered into the rats. The CRF-1 solid feed not containing the compound was freely ingested by the control group, while CRF-1 solid feed containing 0.01333% SNK-860 was freely ingested by the SNK-860 treatment group. At 36 weeks of age and at 52 to 58 weeks of age, the rats were anesthetized and then the eyeballs were excised and used in histopathological examination.

The SNK-860-containing feed was prepared in the following manner. SNK-860 was weighed and mixed well with a small amount of CRF-1 powdered feed. Thereafter, additional powdered feed was added thereto, stirred well and solidified. The dose established this time was 16 mg/kg. The concentration of this agent in the feed, calculated on the basis of the established dose, the weight of the rat and the amount of the feed ingested for 1 day, was 0.01333%.

The histopathological examination was carried out in the following manner. The rat was anesthetized with ether and by intraperitoneal administration of Nembutal, and then the eyeballs were excised. The excised eyeballs were placed in a mixed solution (1 : 1 : 2) consisting of 4% glutaraldehyde, 10% neutral formalin and a phosphate buffer (pH 7.2, 0.3 mol/l). After 60 minutes, the eyeball was cut into halves under a stereoscopic microscope and then stored overnight at 4°C. The next morning, the eyeballs were embedded in a usual manner into paraffin to prepare a transverse section containing a bundle of optic nerves. The section was stained with hematoxylin-eosin.

Fluorescein fundus angiography was carried out by opening the chest under the same anesthesia, injecting fluorescein dextran (Sigma, 50 mg/1 ml PBS) into the heart, and after 5 minutes, excising the eyeballs. That is, the retina was separated from the excised eyeball, spread on a slide glass to prepare a flat mounted specimen of the retina, then observed with a stereoscopic fluorescent microscope and photographed to evaluate retinopathy.

When retinal fold (retinal detachment) accompanied by thickening of the retina around optic nerve head was recognized in the pathological examination, or when fluorescence dye leakage accompanied by retinal vascular tortuosity and/or caliber variation around optic nerve head was recognized in the fluorescein fundus photography, it was assumed that severe retinopathy occurred in the SDT rat.

### 2. Results and Discussion

As duration of the disease was prolonged, retinal vascular tortuosity and/or caliber variation (IRMA: intra-retinal microvascular abnormalities) observed since the stage of human preproliferative diabetic retinopathy, and tractional retinal detachment observed in the stage of proliferative diabetic retinopathy, were exhibited at high degrees in the SDT rats used in evaluation of this agent of the present invention, and thus this agent was administered at the time of from grouping at about 25 weeks of age to 52-58 weeks of age. That is, it was attempted to clarify the inhibitory effect on the onset by setting a longer duration of the disease.

At 36 weeks of age, the onset of tractional retinal detachment was pathologically not recognized in both the groups (both groups: 0/3 eyes (0%)). At 52-58 weeks of age, on the other hand, the degree of onset of tractional retinal detachment or fluorescence dye leakage accompanied by retinal vascular tortuosity and/or caliber variation around optic nerve head, in the untreatment rats, was a high degree (7/11 eyes (64%)), while that of the SNK-860 treatment rats was a low degree (3/14 eyes (21%)). These results show that SNK-860 suppresses severe retinal lesions such as occurrence of IRMA (intra-retinal microvascular abnormalities) and/or tractional retinal detachment in SDT rats. That is, it is revealed that SNK-860 can serve as an agent for suppressing the occurrence or development of severe diabetic retinopathy such as human preproliferative diabetic retinopathy or proliferative diabetic retinopathy, or as a therapeutic agent thereof.

### Industrial Applicability

In the present invention, the effectiveness of the present pharmaceutical preparation against severe diabetic retinopathy is clearly shown by using an animal model exhibiting IRMA (intra-retinal microvascular abnormalities) and tractional retinal detachment. The present invention promises chemotherapy for severe diabetic retinopathy for which no effective medicine was present, that is, preproliferative diabetic retinopathy and proliferative diabetic retinopathy.

## Claims

1. A prophylactic or therapeutic agent for severe diabetic retinopathy, which comprises, as an active ingredient, a compound represented by the general formula: 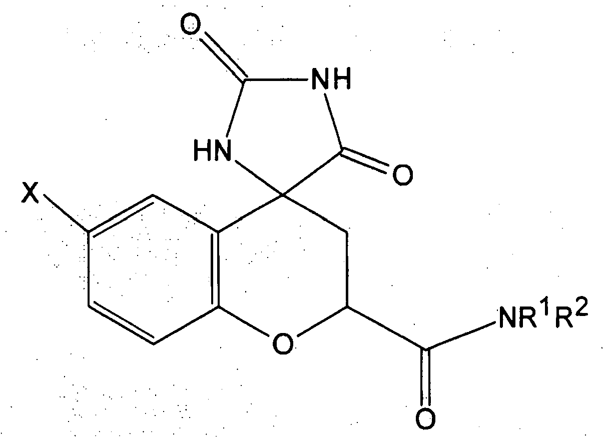 wherein X represents a halogen or a hydrogen atom, R¹ and R² concurrently or differently represent a hydrogen atom or an optionally substituted C1 to C6 alkyl group, or R¹ and R², together with a nitrogen atom bound thereto and optionally another nitrogen atom or an oxygen atom, are combined to form a 5- to 6-membered heterocycle.

2. The prophylactic or therapeutic agent for severe diabetic retinopathy according to claim 1, wherein the compound is (2S,4S)-6-fluoro-2',5'-dioxospiro[chroman-4,4'-imidazolidine]-2-carboxamide.

3. The prophylactic or therapeutic agent for severe diabetic retinopathy according to claim 1, which is used as an agent for suppressing development into preproliferative diabetic retinopathy and/or proliferative diabetic retinopathy or as an agent for suppressing development of preproliferative diabetic retinopathy and/or proliferative diabetic retinopathy.

4. The prophylactic or therapeutic agent for severe diabetic retinopathy according to claim 2, which is used as an agent for suppressing development into preproliferative diabetic retinopathy and/or proliferative diabetic retinopathy or as an agent for suppressing development of preproliferative diabetic retinopathy and/or proliferative diabetic retinopathy.
